# EUROPEAN PATENT APPLICATION

(11) **EP 4 309 747 A1**
(43) Date of publication of application: **24.01.2024**
(21) Application number: 22210835.9
(22) Date of filing: 01.12.2022
(51) Int. Cl.: A63B 21/00, A63B 21/072, A63B 24/00

(54) **DEVICE AND SYSTEM FOR UPPER EXTREMITY REHABILITATION TRAINING BASED ON INFORMATION AND COMMUNICATIONS TECHNOLOGY**

(30) Priority: 18.07.2022 KR 20220087933
(71) Applicant: BioHealthCore Inc., Wonju-si, Gangwon-do 26462 (KR)
(72) Inventor: LEE, Hyo Taek, 48305 Busan (KR)
(74) Representative: Isarpatent

(57) **Abstract**

Disclosed is an upper extremity rehabilitation training device including: a handle shaped body unit which a user is capable of gripping; and an auxiliary module mounting unit configured to couple a gimbal module on which the user terminal is capable of being mounted or one or more auxiliary modules used for upper extremity rehabilitation training. The body unit includes a sensor module and a control module generating user data by measuring a pulse of the user, a grasping power, and a movement of the body unit, and the control module transmits user data generated by the sensor module to the user terminal.

## Description

### TECHNICAL FIELD

The present disclosure relates to a device and a system for upper extremity rehabilitation training based on ICT, and particularly, to a device and a system for upper extremity rehabilitation training, which can provide training contents through a smartphone or a separate display device while measuring user bio information in real time.

### BACKGROUND

As the living environment and economic activity methods change, modern people are extremely short of exercise, and various diseases or physical abnormalities occur due to the lack of exercise.

The exercise equipment using conventional information technology is mostly devices for measuring and checking the amount of exercise in the process of performing ordinary running exercises or walking exercises and transmitting the amount of exercise to applications of smartphones to record or store exercise amount. There is a lack of specialized core exercise equipment and health care contents, and infrastructure for strengthening the core muscles needed for high-age groups or pregnant women who cannot perform strenuous exercise due to the body structure.

In particular, as the elderly health problems caused by the recent aging are emerging as a social problem, an old-friendly exercise contents or exercise assistance device is required, in which the age group can strengthen the core muscle power without straining the body when exercising, and continuously provides systematic customized exercise information in the exercise process and gains health.

In particular, there is a problem with the lack of professionalism in that exercise equipment for rehabilitation and stabilization of upper extremity joints can only be simple repetitive movements, which does not provide optimized movements according to body shape, and it is difficult to control, evaluate, and examine the exact posture of patients or rehabilitation.

Therefore, an object of the present disclosure is to provide an upper extremity rehabilitation training program optimized to a rehabilitation target through providing a multi-ICT convergence healthcare controller or upper extremity rehabilitation training device that synchronizes with smart device VR/AR contents of the health device-based aerobic/anaerobic oxygen complex exercise, living sports and silver rehabilitation.

### SUMMARY

The present disclosure has been made in an effort to provide an upper extremity rehabilitation training program optimized to a rehabilitation target through an upper extremity rehabilitation training device which can interlock with a smartphone and a separate auxiliary device.

However, a technical object to be achieved by the exemplary embodiment of the present disclosure is not limited to the technical object and there may be other technical objects.

An exemplary embodiment of the present disclosure provides an upper extremity rehabilitation training device including: a handle shaped body unit which a user is capable of gripping; and an auxiliary module mounting unit configured to couple a gimbal module on which the user terminal is capable of being mounted or one or more auxiliary modules used for upper extremity rehabilitation training. The body unit includes a sensor module and a control module generating user data by measuring a pulse of the user, a grasping power, and a movement of the body unit, and the control module transmits user data generated by the sensor module to the user terminal.

Alternatively, the sensor modules may include at least one of a grasping power measurement sensor capable of measuring the grasping power of the user, a pulse measurement sensor generating pulse data by receiving a pulse signal of the user, a speed sensor, a gyro sensor, a geomagnetic sensor, a location sensor, and a vibration module.

Alternatively, the auxiliary module may further include at least one of a weight additional module adding a predetermined weight for exercise of muscle, a racket module for racket exercise, a jump rope module, a treadmill module, and a climbing stick module.

Alternatively, the control module may deliver movement data including a movement distance, a direction, a speed, and an acceleration to the user terminal in X, Y, and Z-axis directions in real time with movement of the body unit.

Alternatively, the control module may determine whether the user grips the body unit and whether to initiate rehabilitation training based on user data generated by the sensor module, and deliver pulse numerical data and grasping power numerical data of the user to the user terminal.

Alternatively, the upper extremity rehabilitation training device may further include a display module reproducing upper extremity rehabilitation training contents delivered from the control module.

Alternatively, the body unit may further include a vibration module, the vibration module may deliver a vibration signal to the user based on a vibration signal generated by the control module, and the vibration signal may be used for guiding the upper extremity rehabilitation training of the user.

Another exemplary embodiment of the present disclosure provides an upper extremity rehabilitation training system including: an upper extremity rehabilitation training device; a user terminal mountable on the upper extremity rehabilitation training device; and a server. The upper extremity rehabilitation training device may include a handle shaped body unit which a user is capable of gripping, and an auxiliary module mounting unit configured to couple a gimbal module on which the user terminal is capable of being mounted or one or more auxiliary modules used for upper extremity rehabilitation training, the body unit may include a sensor module and a control module generating user data by measuring a pulse of the user, a grasping power, and a movement of the body unit, and the control module may transmit user data generated by the sensor module to the user terminal or the server.

According to an exemplary embodiment of the present disclosure, multiple sports or training programs optimized to a rehabilitation target can be performed through an upper extremity rehabilitation training system, and a feedback can be received in real time.

Further, basic information for exercise enhancement can be provided through multiple sensors of an upper extremity rehabilitation training device, and various effects can be implemented such as making feeling an interest in an exercise through various upper extremity rehabilitation training contents.

The foregoing summary is illustrative only and is not intended to be in any way limiting. In addition to the illustrative aspects, embodiments, and features described above, further aspects, embodiments, and features will become apparent by reference to the drawings and the following detailed description.

### BRIEF DESCRIPTION OF THE DRAWINGS

FIG. 1 is a block diagram illustrating a configuration of an upper extremity rehabilitation training system according to an exemplary embodiment of the present disclosure.
FIG. 2 is a block diagram illustrating a configuration of an upper extremity rehabilitation training device according to an exemplary embodiment of the present disclosure.
FIG. 3 is a block diagram illustrating a hardware configuration of the upper extremity rehabilitation training device according to an exemplary embodiment of the present disclosure.
FIGS. 4A to 4D are diagrams for describing the upper extremity rehabilitation training device according to an exemplary embodiment of the present disclosure.
FIG. 5 is a diagram for describing the upper extremity rehabilitation training device according to an exemplary embodiment of the present disclosure.
FIG. 6 is a diagram for describing the upper extremity rehabilitation training system according to an exemplary embodiment of the present disclosure.

### DETAILED DESCRIPTION

In the following detailed description, reference is made to the accompanying drawing, which forms a part hereof. The illustrative embodiments described in the detailed description, drawing, and claims are not meant to be limiting. Other embodiments may be utilized, and other changes may be made, without departing from the spirit or scope of the subject matter presented here.

Hereinafter, exemplary embodiments of the present disclosure will be described in detail so as to be easily implemented by those skilled in the art, with reference to the accompanying drawings. However, the present disclosure may be modified in various different ways, all without departing from the spirit or scope of the present disclosure. In addition, in the drawings, in order to clearly describe the present disclosure, a part not related to the description is not omitted and like reference numerals designate like elements throughout the specification.

Throughout the specification, when it is described that a part is "connected" with another part, it means that the part may be "directly connected" with the another part and the parts may be "electrically or mechanically connected" to each other with still another element interposed therebetween. Further, when a part "includes" a component, it means that other components may be further included, rather than excluding other components, unless otherwise stated, and it is to be understood that the existence or addition of one or more other features, numbers, steps, operations, components, parts, or combinations thereof is not precluded in advance.

In the present disclosure, 'terminal' may be a wireless communication device in which portability and mobility are guaranteed, and may be, for example, all types of handheld based wireless communication devices such as a smartphone, a tablet PC, or a laptop. Further, 'terminal' can also be a wired communication device such as a PC which can access another terminal or server through a network. Further, the network means a connection structure in which information can be exchanged between respective nodes such as terminals and servers, and includes a local area network (LAN), a wide area network (WAN), a world wide web (WWW), a wired/wireless data communication network, a telephone network, a wired/wireless television communication network, etc.

An example of a wireless data communication network includes 3G, 4G, 5G, 3rd Generation Partnership Project (3GPP), Long Term Evolution (LTE), World Interoperability for Microwave Access (WIMAX), Wi-Fi, Bluetooth communication, infrared communication, ultrasonic communication, Visible Light Communication (VLC), LiFi, etc., but is not limited thereto.

The following exemplary embodiment is a detailed description to help understand the present disclosure, and is not limited to the scope of the present disclosure. Therefore, the present disclosure of the same range of performing the same function as the present disclosure will also belong to the scope of the present disclosure.

Further, each configuration, procedure, process, or a method included in each exemplary embodiment of the present disclosure may be shared within a range without technical mutual contradiction.

Hereinafter, an exemplary embodiment of the present disclosure will be described in detail with reference to the accompanying drawings.

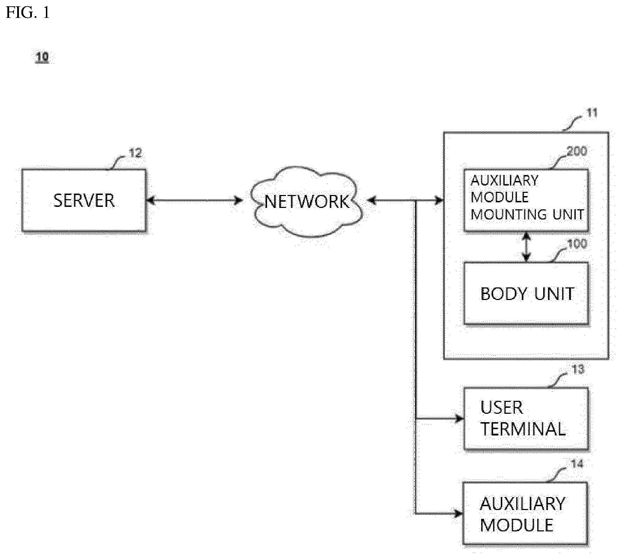

FIG. 1 is a block diagram illustrating a configuration of an upper extremity rehabilitation training system according to an exemplary embodiment of the present disclosure.

The upper extremity rehabilitation training system 10 includes an upper extremity rehabilitation training device 11, a server 12, a user terminal 13, and an auxiliary module 14. They may be connected through a network or various communication interfaces. The upper extremity rehabilitation training device 11 may include a body unit 100 and an auxiliary module mounting unit 200. The auxiliary module mounting unit 200 may be a device for mounting the user terminal 13, and may be user terminal cradle coupled to a gimbal device. The user terminal 13 may include a portable terminal such as the smartphone, and the tablet PC. The auxiliary module 14 may be an auxiliary device for performing various types of upper extremity rehabilitation trainings such as a weight, a climbing pole, and a baseball bat.

An upper extremity rehabilitation training method may be executed by the upper extremity rehabilitation training device 11, the server 12, or the user terminal 13. For example, the upper extremity rehabilitation training method may execute upper extremity rehabilitation contents or an upper extremity rehabilitation program, guide the upper extremity rehabilitation training to a user through a user interface, and display a rehabilitation exercise result and measurement data.

The upper extremity rehabilitation training system 10 may transmit data generated by the upper extremity rehabilitation training device 11 to the user terminal 13, analyze the data in the user terminal, and then display the data to the user, and transmit the data to the server 12 in the user terminal 13, and then process the data in the server 12 and also deliver the data to the user terminal 13 again.

The upper extremity rehabilitation training device 11 and the user terminal 13 may transmit and receive the data by using short-range wireless communication such as Bluetooth and Zigbee or a wired communication protocol through the direct connection.

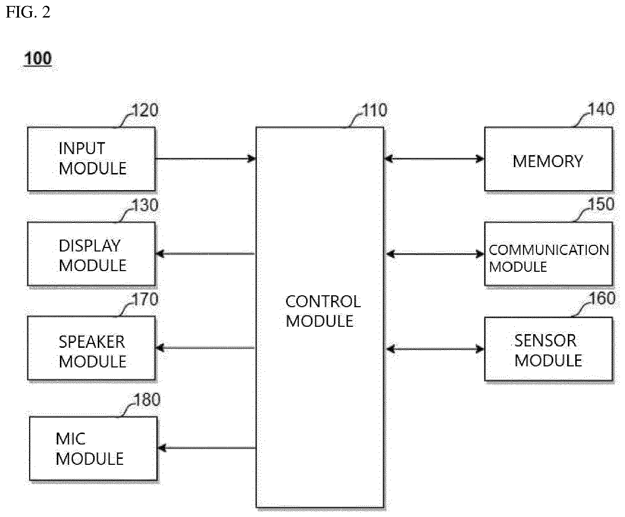

FIG. 2 is a block diagram illustrating a configuration of an upper extremity rehabilitation training device according to an exemplary embodiment of the present disclosure.

As illustrated in FIG. 2, the upper extremity rehabilitation training device or the body unit 100 of the upper extremity rehabilitation training device may include a control module 110, an input module 120, a display module 130, a memory 140, a communication module 150, a sensor module 160, a speaker module 170, and a mic module 180.

The communication module 150 may communicate the data with each of the body unit 100 of the upper extremity rehabilitation training device and the user terminal or the server. The communication module 150 may be a device including hardware or software required for transmitting and receiving a signal such as a control signal or a data signal through wired/wireless connection with another network device. The communication module 150 may provide a communication interface by implementing a wired communication function using serial communication and a wireless communication function using a communication network such as Bluetooth, and WiFi. The communication module 140 may provide various interface schemes including a Bluetooth scheme, a CDMA scheme or a Zigbee scheme, a power line communication scheme, a serial communication scheme, a DMX-512 scheme, a USB scheme, a CAN scheme, etc.

The upper extremity rehabilitation training program may be stored in the memory 140. The upper extremity rehabilitation training program may generate a vibration signal for processing various sensor data generated while the upper extremity rehabilitation training program is performed or guiding the upper extremity rehabilitation training. The upper extremity rehabilitation training program may be stored in at least one of the body unit of the upper extremity rehabilitation training device, the user terminal, or the server.

The memory 140 stores an operating system for driving the upper extremity rehabilitation training device or various types of data generated in the process of executing the upper extremity rehabilitation training program.

In this case, the memory 140 is collectively referred to as a non-volatile storage device continuously holding stored information even though power is not supplied and a volatile storage device requiring power in order to hold the stored information.

Further, the memory 140 may perform a function of temporarily or permanently storing data processed by the control module 110. Here, the memory 140 may include a magnetic storage media or a flash storage media in addition to the volatile storage device requiring the power in order to hold the stored information, but the scope of the present disclosure is not limited thereto.

The control module 110 executes a program stored in the memory 140, and may control an overall process according to the execution of the upper extremity rehabilitation training program.

The control module 110 may include all types of devices capable of processing data. For example, the control module 110 may mean a data processing device embedded in hardware, which has a physically structured circuit in order to perform a function expressed by a code or a command included in the program. An example of the data processing device embedded in the hardware may include a processing device such as a Microprocessor, a Central Processing Unit (CPU), a Processor core, a Multiprocessor, an Application-Specific Integrated Circuit (ASIC), and A Field Programmable Gate Array (FPGA), but the scope of the present disclosure is not limited thereto.

The sensor module 160 may include an acceleration sensor, a geomagnetic sensor, a gyro sensor, etc., for measuring a movement related to the upper extremity rehabilitation training of the user. The sensor module 160 may include a heart rate sensor, a pressure sensor, etc., for determining a state of the user. The pressure sensor is attached to the body unit of the upper extremity rehabilitation training device to be used for measuring grasping power when the user grasps the upper extremity rehabilitation training device.

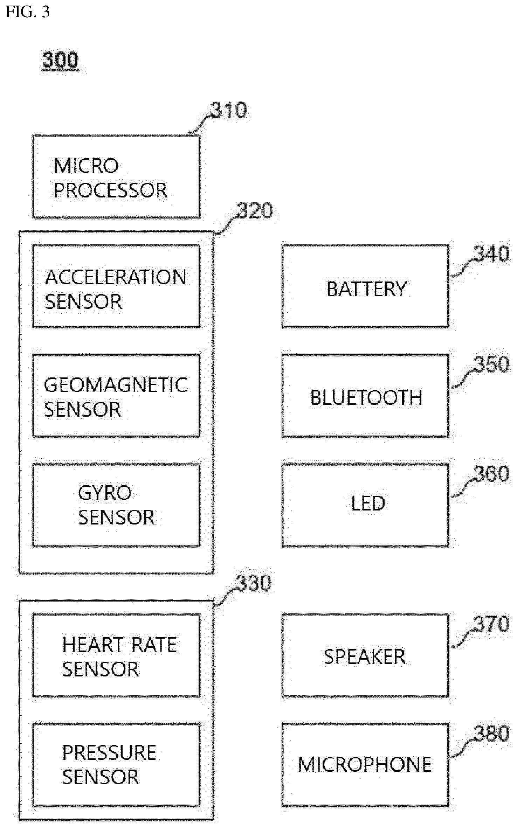

FIG. 3 is a block diagram illustrating a hardware configuration of the upper extremity rehabilitation training device according to an exemplary embodiment of the present disclosure.

As illustrated in FIG. 3, the upper extremity rehabilitation training device 300 may include a microprocessor 310, a first sensor module 320, a second sensor module 330, a battery 340, a Bluetooth 350, an LED 360, a speaker 370, and a microphone 380. The first sensor module 320 may be an inertial measurement unit (IMU), and include the acceleration sensor, the geomagnetic sensor, and the gyro sensor. The second sensor module 330 may include the heart rate sensor and the pressure sensor.

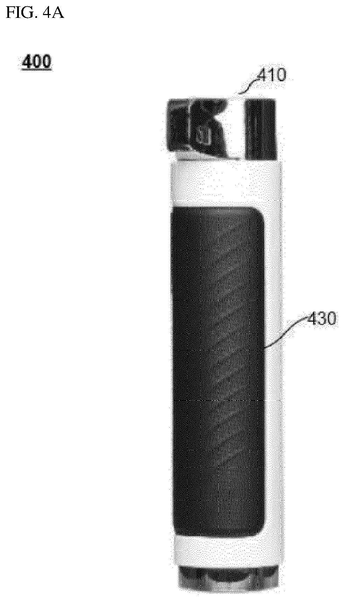

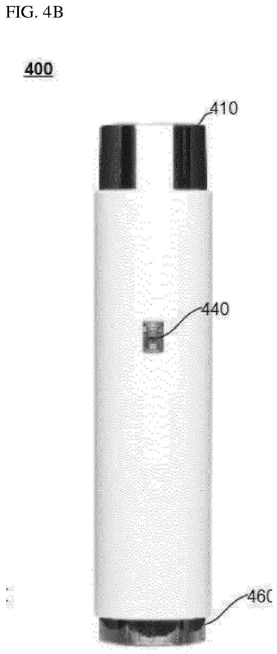

FIGS. 4A and 4B are diagrams for describing the upper extremity rehabilitation training device according to an exemplary embodiment of the present disclosure.

Referring to FIG. 4A, an upper surface 410 of the upper extremity rehabilitation training device 400 may be configured to mount a gimbal type cradle capable of holding the user terminal or an auxiliary module used for the upper extremity rehabilitation training such as the weight, and the climbing stick. The upper extremity rehabilitation training device 400 may include a cylindrical or handle type body unit easily gripped by the user. The upper extremity rehabilitation training device 400 may further include a grasping power measurement unit 430 in the handle type body unit in order for the user to accurately grip a sensor module for measuring the grasping power or the pressure.

Referring to FIG. 4B, the upper extremity rehabilitation training device 400 may further include a pulse measurement sensor 440 for measuring a pulse of the user and a charging terminal 460 for charging the upper extremity rehabilitation training device 400 or a battery included in the upper extremity rehabilitation training device 400. The charging terminal 460 may include various types of charging terminals such as a micro USB 5-pin terminal, and a C type terminal.

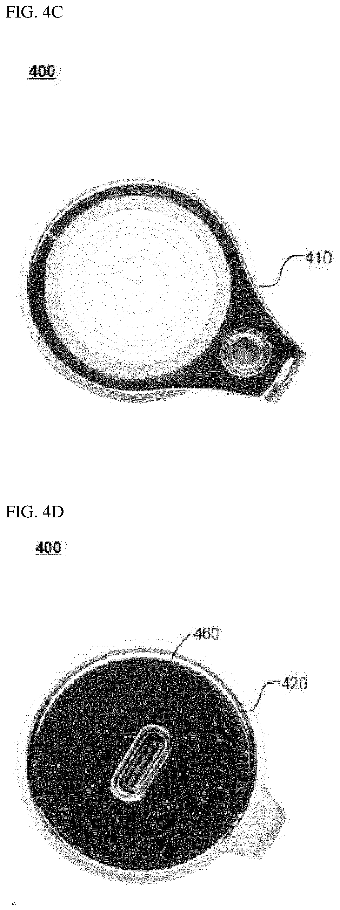

FIG. 4C is a diagram illustrating the upper surface 410 of the upper extremity rehabilitation training device 400.

A power button for initiating an operation of the upper extremity rehabilitation training device 400 and a groove on which the auxiliary module used for the upper extremity rehabilitation training, such as the weight, and the climbing stick, may be located on the upper surface 410.

FIG. 4D is a diagram illustrating a lower surface 420 of the upper extremity rehabilitation training device 400. The lower surface 420 of the upper extremity rehabilitation training device 400 may also be configured to mount the auxiliary module used for the upper extremity rehabilitation training, such as the weight, and the climbing stick, like the upper surface 410. The charging terminal 460 for charging the battery included in the upper extremity rehabilitation training device 400 may be located on the lower surface 420. The charging terminal 460 may include various types of charging terminals such as a micro USB 5-pin terminal, and a C type terminal.

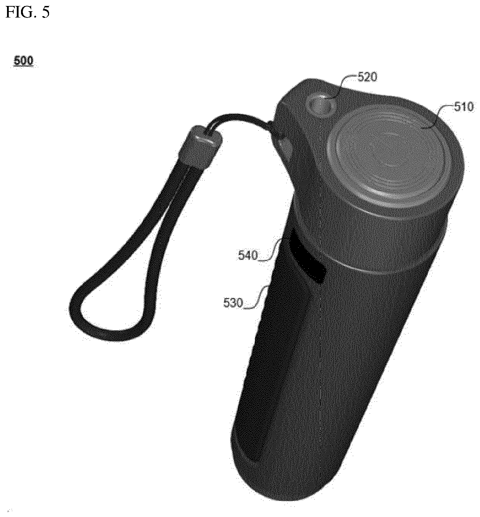

FIG. 5 is a diagram for describing the upper extremity rehabilitation training device according to an exemplary embodiment of the present disclosure.

As illustrated in FIG. 5, the upper extremity rehabilitation training device 500 may include a power button 510 on an upper surface. The power button 510 may include an LED module around the power button 510. The upper extremity rehabilitation training device 500 may include a camera tripod hole 520. Various types of auxiliary modules for the upper extremity rehabilitation training such as the weight, a fishing module, a badminton module, and a baseball module, is detachable from/attachable to the camera tripod hole 520.

The upper extremity rehabilitation training device 500 may include a pressure sensor 530 and a pulse sensor 540. The pressure sensor 530 or a grasping power sensor may be formed at a handle position by considering a grip area according to a user body type. The upper extremity rehabilitation training device 500 may measure the grasping power of the user through the pressure sensor 530.

Meanwhile, if the pressure sensor 530 is a device capable of measuring the pressure, the pressure sensor 530 may be applied to the present disclosure. The pressure sensor 530 may be a commercialized circular and rectangular form, and force may be measured by the pressure sensor 530 at the time of gripping the upper extremity rehabilitation training device 500. An external surface of the pressure sensor 530 may be made of a soft material.

The upper extremity rehabilitation training device 500 may generate whether to recognize a pulse, pulse numerical data, battery voltage, grasping power numerical data, X-left/right surface angle data, Y front/rear surface angle data, etc, and transmit the data to the user terminal or the server.

The upper extremity rehabilitation training device 500 may include a connection groove for connecting a smart grip gimbal type device. The upper extremity rehabilitation training device 500 may further include a vibration module for vibration feedback.

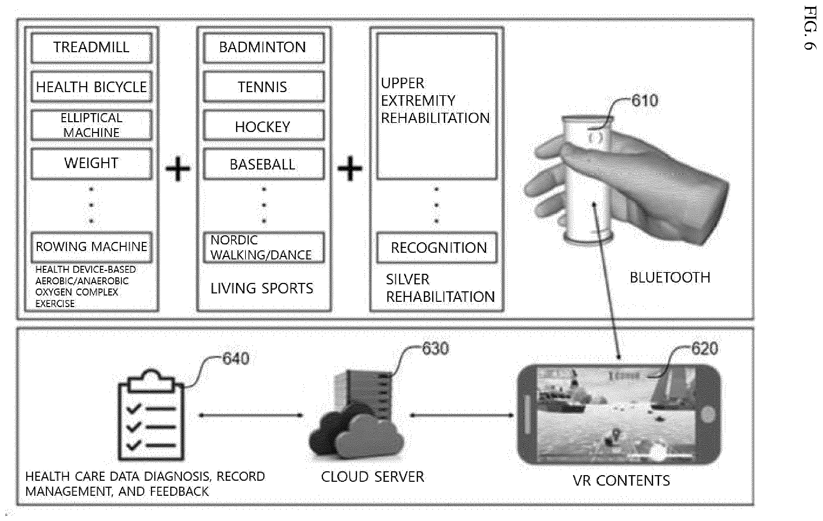

FIG. 6 is a diagram for describing the upper extremity rehabilitation training system according to an exemplary embodiment of the present disclosure.

The upper extremity rehabilitation training system 600 may be connected to a user terminal 620 by using the Bluetooth or various types of wired/wireless short-range communication. The upper extremity rehabilitation training system may provide various contents and programs for the upper extremity rehabilitation training such as VR contents through the user terminal 620 including the smartphone. The upper extremity rehabilitation training system may deliver data generated by the upper extremity rehabilitation training device 600 or the user terminal 620 to a cloud server 630, and analyze and manage the data in the cloud server 630. The upper extremity rehabilitation training system may reflect analysis/feedback for separate healthcare data, diagnosis data of the user, and accumulated data, etc., on the cloud server 630.

The upper extremity rehabilitation training system may provide a health device-based aerobic/anaerobic oxygen complex service such as a treadmill, a health bicycle, an elliptical machine, and a weight, to the user. The upper extremity rehabilitation training system may provide a living sport program such as a badminton, a tennis, a hockey, a baseball, a Nordic walking, and a dance, to the user. For example, the upper extremity rehabilitation training system may provide fishing contents by utilizing the pressure sensor and the vibration sensor. The upper extremity rehabilitation training system may provide a silver rehabilitation program such as upper extremity rehabilitation, and recognition, to the user.

Functions implemented inside and outside a body of the upper extremity rehabilitation training device are described below. There is an accessory connection portion at an upper end of the body, and the body may be connected to a self camera rod or the weight of the user through the accessory connection portion.

A power button capable of performing an on/off function of the body may be provided at one outer side of the body, a user motion and bio information may be tracked through an internal configuration and an algorithm to synchronize a game and a health care program with each other.

The upper extremity rehabilitation training device includes a separate LED to perform battery level display and light functions, and implement a sound function through a speaker/microphone.

Further, the upper extremity rehabilitation training device may include a separate battery charging unit. Specifically, a micro USB 5 PIN may be used. The upper extremity rehabilitation training device may connect a Nordic pole or a weight for a ski through the accessory connection portion.

The upper extremity rehabilitation training device may include an IMU sensor that senses a movement of the user by enabling gyro, acceleration, geomagnetic measurement inside the body.

Further, the upper extremity rehabilitation training device may include a bio sensor that is capable of measuring the pulse and the grasping power of the user who uses the corresponding device, and senses the bio information of the user.

The upper extremity rehabilitation training device may include a wireless battery which is chargeable, and include an LED module including the battery level and light functions. The upper extremity rehabilitation training device may include a speaker and a microphone including a sound playback and AI speaker functions.

Further, the upper extremity rehabilitation training device may include a Bluetooth function for synchronizing the VR/AR contents within a smart device possessed by the user. In this case, an application installed in the smart device may include the health device-based aerobic/anaerobic oxygen complex exercise such as the treadmill, the health bicycle, the elliptical machine, the weight, and a rowing machine, living sports such as the badminton, the tennis, the hockey, the baseball, the Nordic walking, and the dance, an exercise program (automatically setting a difficulty level by determining the movement of the user) of silver rehabilitation for the upper extremity rehabilitation and recognition (including five elements), and game based VR/AR contents. The VR/AR contents may sense an angular speed which rotates to x, y, and z axes from the gyro sensor and a speed which is changed for the x, y, and z axes from the acceleration sensor.

For example, the upper extremity rehabilitation training device may sense outdoor orientation data for the x, y, and z axes from the geomagnetic sensor, and synchronize data by measuring bio data (an outer side and both sides) of the user from the heart rate sensor and the pressure sensor.

An upper case of the upper extremity rehabilitation training device may be detachable, and include a function for connecting a self camera rod used for using a smart device camera function, the weight for a dumbbell exercise, an accessory such as living sport equipment such as a golf, the tennis, the badminton, the hockey, and the baseball, which is capable of performing a function such as an exercise trajectory, and a posture analysis.

A lower case of the upper extremity rehabilitation training device may be detachable, and include a function of connecting accessories such as the weight for the dumbbell exercise, the Nordic pole, and various other health care equipment (detachable from a mechanism with a handle, such as the treadmill, an ergometer, the elliptical machine, and the rowing machine.).

A surface of the upper extremity rehabilitation training device may include the power button, a heart rate measurement unit, a button for smart device camera and light functions, and the battery charging unit.

Further, an exercise such as the baseball installed in an application is enabled by using the upper extremity rehabilitation training device or the upper extremity rehabilitation training system, and in this case, a trajectory of a bat is displayed, and information such as a bat speed, Peak Hand Speed, Attack Angle, and Time to Contact may be displayed, of course, and furthermore, an entire bat swing motion may be displayed, and in this case, factor information such as separate On Plane, and Blast Factor, may also be displayed.

An exemplary embodiment of the present disclosure may be implemented even in the form of a recording medium including a command executable by a computer such as a program module executed by the computer. The recording medium may include a computer readable medium, and the computer readable medium may be a predetermined available medium accessible by the computer or includes all of volatile and non-volatile media and removable and irremovable media. Further, the computer readable storage medium includes a computer storage medium, and a computer storage medium includes all of the volatile and non-volatile and removable and irremovable media implemented by a predetermined method or technology for storing information such as a computer readable command, a data structure, a program module, or other data.

The aforementioned description of the present disclosure is used for exemplification, and it may be understood by those skilled in the art that the present disclosure may be easily modified in other detailed forms without changing the technical spirit or requisite features of the present disclosure. Therefore, it should be appreciated that the aforementioned embodiments are illustrative in all aspects and are not restricted. For example, respective constituent elements described as single types may be distributed and implemented, and similarly, constituent elements described to be distributed may also be implemented in a coupled form.

The scope of the present disclosure is represented by claims to be described below rather than the detailed description, and it is to be interpreted that the meaning and scope of the claims and all the changes or modified forms derived from the equivalents thereof come within the scope of the present disclosure.

From the foregoing, it will be appreciated that various embodiments of the present disclosure have been described herein for purposes of illustration, and that various modifications may be made without departing from the scope and spirit of the present disclosure. Accordingly, the various embodiments disclosed herein are not intended to be limiting, with the true scope and spirit being indicated by the following claims.

## Claims

1. An upper extremity rehabilitation training device comprising:
a handle shaped body unit which a user is capable of gripping; and
an auxiliary module mounting unit configured to couple a gimbal module on which a user terminal is capable of being mounted or one or more auxiliary modules used for an upper extremity rehabilitation training,
wherein the body unit includes a sensor module and a control module generating user data by measuring a pulse of the user, a grasping power, and a movement of the body unit, and
the control module transmits user data generated by the sensor module to the user terminal.

2. The upper extremity rehabilitation training device of claim 1, wherein the sensor modules includes at least one of a grasping power measurement sensor capable of measuring the grasping power of the user, a pulse measurement sensor generating pulse data by receiving a pulse signal of the user, a speed sensor, a gyro sensor, a geomagnetic sensor, and a location sensor.

3. The upper extremity rehabilitation training device of claim 1, wherein the auxiliary module further includes at least one of a weight additional module adding a predetermined weight for exercise of muscle, a racket module for racket exercise, a jump rope module, a treadmill module, and a climbing stick module.

4. The upper extremity rehabilitation training device of claim 1, wherein the control module delivers movement data including a movement distance, a direction, a speed, and an acceleration to the user terminal in X, Y, and Z-axis directions in real time with movement of the body unit.

5. The upper extremity rehabilitation training device of claim 1, wherein the control module determines whether the user grips the body unit and whether to initiate rehabilitation training based on user data generated by the sensor module, and delivers pulse numerical data and grasping power numerical data of the user to the user terminal.

6. The upper extremity rehabilitation training device of claim 1, further comprising:
a display module reproducing upper extremity rehabilitation training contents delivered from the control module.

7. The upper extremity rehabilitation training device of claim 1, wherein the body unit further includes a vibration module,
the vibration module delivers a vibration signal to the user based on a vibration signal generated by the control module, and
the vibration signal is used for guiding the upper extremity rehabilitation training of the user.

8. The upper extremity rehabilitation training device of claim 1, wherein the control module provides rehabilitation training contents for the upper extremity rehabilitation training of the user through the user terminal, and
when the user conducts rehabilitation training according to the rehabilitation training contents, at least one of a muscular strength, flexibility, and a body operation range of the user is measured based on the user data generated by the sensor module, and an upper extremity rehabilitation training target and an upper extremity rehabilitee training strength are determined and reflected on the rehabilitation training contents.

9. An upper extremity rehabilitation training system comprising:
an upper extremity rehabilitation training device;
a user terminal mountable on the upper extremity rehabilitation training device; and
a server,
wherein the upper extremity rehabilitation training device includes a handle shaped body unit which a user is capable of gripping, and an auxiliary module mounting unit configured to couple a gimbal module on which the user terminal is capable of being mounted or one or more auxiliary modules used for upper extremity rehabilitation training,
the body unit includes a sensor module and a control module generating user data by measuring a pulse of the user, a grasping power, and a movement of the body unit, and
the control module transmits user data generated by the sensor module to the user terminal or the server.
